# EUROPÄISCHE PATENTSCHRIFT

(11) **EP 1 148 117 B1**
(45) Veröffentlichungstag und Bekanntmachung des Hinweises auf die Patenterteilung: **24.03.2004**
(21) Anmeldenummer: 01109628.6
(22) Anmeldetag: 18.04.2001
(51) Int. Cl.: C11D 3/39, C07C 251/24, C07F 9/30

(54) **Bleichaktive Dendrimer-Liganden und deren Metall-Komplexe**
Bleach activating dendrimer ligands and their metal complexes
Ligands dendrimériques comme activateurs de blanchiment et leurs complexes avec des métaux

(30) Priorität: 20.04.2000 DE 10019878
(43) Veröffentlichungstag der Anmeldung: 24.10.2001
(73) Patentinhaber: Clariant GmbH, 65929 Frankfurt am Main (DE)
(72) Erfinder: Fischer, Claudia, Dr., 65835 Liederbach (DE); Issberner, Jörg, Dr., 47804 Krefeld (DE); Vögtle, Fritz, Prof. Dr., 53347 Alfter-Impekoven (DE)
(74) Vertreter: Güthlein, Paul

(56) Entgegenhaltungen:
- WO-A-01/05925
- DE-A- 19 621 510
- US-A- 2 928 876
- MATSUMOTO N ET AL: "SYNTHESIS AND CHARACTERIZATION OF A CHLORO-BRIDGED BINUCLEAR IRON(III) COMPLEX" BULLETIN OF THE CHEMICAL SOCIETY OF JAPAN,JP,JAPAN PUBLICATIONS TRADING CO. TOKYO, Bd. 58, Nr. 12, 1. Dezember 1985 (1985-12-01), Seiten 3621-3622, XP002037382 ISSN: 0009-2673
- MOORS R ET AL: "DENDRIMERE POLYAMINE. DENDRIMERIC POLYAMINES" CHEMISCHE BERICHTE,DE,VERLAG CHEMIE GMBH. WEINHEIM, Bd. 126, 1993, Seiten 2133-2135, XP002037381 ISSN: 0009-2940
- ISSBERNER J ET AL: "Poly(amine/imine) Dendrimers Bearing Planar Chiral Terminal Groups - Synthesis and Chiroptical Properties" TETRAHEDRON: ASYMMETRY,NL,ELSEVIER SCIENCE PUBLISHERS, AMSTERDAM, Bd. 7, Nr. 8, 1. August 1996 (1996-08-01), Seiten 2223-2232, XP004048278 ISSN: 0957-4166
- PATENT ABSTRACTS OF JAPAN vol. 1999, no. 05, 31. Mai 1999 (1999-05-31) -& JP 11 050096 A (LION CORP), 23. Februar 1999 (1999-02-23)
- BARBERA ET AL: "Copper-containing dendromesogens: the influence of the metal on the mesomorphism" LIQUID CRYSTALS, Bd. 27, Nr. 2, Februar 2000 (2000-02), Seiten 255-262, XP001007487 ISSN: 0267-8292

## Beschreibung

Es ist bekannt, dass sich das Bleichvermögen peroxidischer Bleichmittel, wie Wasserstoffperoxid, Perboraten, Percarbonaten, Persilikaten und Perphosphaten, in Wasch- und Reinigungsmitteln und somit die Effizienz dieser Bleichmittel zur Entfernung von Tee-, Kaffee-, Obst- oder Rotweinflecken erst bei höheren Temperaturen von deutlich über 60°C voll entfaltet. Zur Verbesserung der bei niedrigeren Temperaturen, vor allem unter 60°C, stark herabgesetzten Bleichwirkung können Verbindungen zur Aktivierung der Peroxidbleichmittel eingesetzt werden. Für diesen Zweck wurden eine Reihe von Übergangsmetallsalzen bzw. entsprechende Komplexe mit meist chelatisierenden Verbindungen vorgeschlagen, doch ist die Wirksamkeit eines Metalls bzw. einer speziellen Kombination von Übergangsmetall und Komplexligand nicht voraussagbar.

In einer Vielzahl von Schriften, beispielsweise in WO 96/06154 und EP 458 397 werden Metall-Komplexe mit einem hohen Aktivierungspotential beansprucht. In DE 1 980 9713 werden Übergangsmetallkomplexe mit Polyamidoamindendrimeren-Ligandsystemen beschrieben.
Zielsetzung ist, Bleichkatalysatoren zu finden, die ein hohes Oxidations- und Bleichvermögen zeigen und gleichzeitig die Farben gefärbter Textilien oder Oberflächen, sowie die Textilfasern möglichst wenig schädigen.

Ein dendritisches Polyamin und sein Cobalt-Komplex sind in Chem. Ber. 1993, S.2133-2135 beschrieben. DE-A-196 21510 beschreibt Dendrimere mit planarchiralen oder axial-chiralen Endgruppen.

US-A-2 928 876 beschreibt die Verbindung N,N,N',N'-Tetrakis-(alphamethylsalicyliden-2-aminoethyl)-ethylendiamin und die analoge 2-amino-propyl-Verbindung. Die erstgenannte Verbindung ist auch in BULLETIN OF THE CHEMICAL SOCIETY OF JAPAN, Bd. 58, Nr. 12, 1. Dezember 1985, Seiten 3621-3622, beschrieben. Poly(amine/imine) Dendrimers Bearing Planar Chiral Terminal Groups - Synthesis and Chiroptical Properties' TETRAHEDRON: ASYMMETRY, Bd. 7, Nr. 8, 1. August 1996, Seiten 2223-2232, beschreibt Polyamin-Dendrimere, die als Endgruppen solche enthalten, die sich von 5-Formyl-4-hydroxy[2.2]paracyclophan ableiten. In JP 11 050 096 werden granulare Reinigungsmittel beschrieben, die Metallkomplexe enthalten, wobei der Ligand dieser Metallkomplexe drei oder mehr koordinierende Stickstoffatome enthält. LIQUID CRYSTALS, Bd. 27, Nr. 2, Februar 2000, Seiten 255-262, beschreibt Poly(ethylenimin)-Dendrimere, deren Endgruppen sich ableiten von 4-(4-Alkoxybenzoyloxy)salicylaldehyd.

Es wurde nun gefunden, dass Übergangsmetallkomplexe mit Dendrimeren vom Polyalkylenimintyp, die Bleichwirkung der Persauerstoffverbindungen beim Bleichen von gefärbten Anschmutzungen sowohl an Textilien wie auch an harten Oberflächen verbessern ohne Farben und Fasern zu zerstören. Darüber hinaus wurde gefunden, dass der Einsatz von Dendrimeren, die nicht komplex mit Übergangsmetallen gebunden sind, in Wasch- und Reinigungsmitteln das Oxidations- und Bleichvermögen der Mittel in wässriger Lösung verstärken.

Gegenstand der Erfindung sind Verbindungen der Formel I

(R¹R¹)N - X - N(R¹R¹) (I)

worin
R¹ eine Gruppe der Formel (R²R³)N-(CH₂)ₙ-,
R² und R³ jeweils eine Gruppe der Formel (R⁴R⁵)N-(CH₂)ₙ-, n die Zahlen 2 oder 3, oder R² und R³ zusammen die Gruppe der Formel A oder R² Wasserstoff und R³ eine Gruppe der Formel 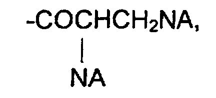
R⁴ und R⁵ jeweils eine Gruppe der Formel (R⁶R⁷)-N-(CH₂)ₙ-, n die Zahlen 2 oder 3 oder R⁴ und R⁵ zusammen die Gruppe der Formel A oder R⁴ Wasserstoff und R⁵ eine Gruppe der Formel 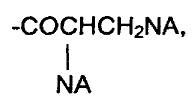
R⁶ und R⁷ jeweils eine Gruppe der Formel (R⁸R⁹)N-(CH₂)ₙ-, n die Zahlen 2 oder 3 oder R⁶ und R⁷ zusammen die Gruppe der Formel A oder R⁸ Wasserstoff und R⁹ eine Gruppe der Formel 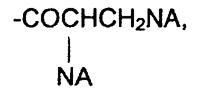
R⁸ und R⁹ zusammen die Gruppe der Formel A oder
R⁸ Wasserstoff und R⁹ eine Gruppe der Formel 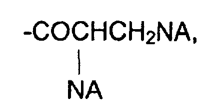
A eine Gruppe der Formel 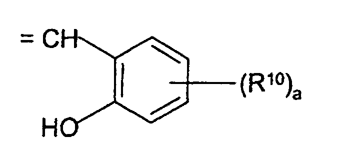
bedeutet, worin a eine ganze Zahl von 1 bis 4 und R¹⁰ Wasserstoff, C₁-C₃₀-Alkyl-, Cycloalkyl- oder Arylreste, C₁-C₄-Alkoxygruppen, substituierte oder unsubstituierte Amino- bzw. Ammoniumgruppen, Halogenatome, Sulfogruppen, Carboxylgruppen oder Gruppierungen der Formel -(CH₂)ᵣ-COOH, -(CH₂)ᵣ-SO₃H, -(CH₂)ᵣ-PO₃H₂, -(CH₂)ᵣ-OH bedeuten, wobei r eine ganze Zahl von 0 bis 4 bedeutet und die genannten Säuregruppen auch in Salzform vorliegen können, und X eine Gruppe der Formeln
-(CH₂)ₙ-, -(CH₂)₃-NR¹¹-(CH₂)₃-, -(CH₂)₂-NR¹¹-(CH₂)₂-, C₂-C₂₀-Alkylen, -(CH₂)ₗ-[O-(CH₂)ₖ]ₘ-O-CH₂)ₗ)- bedeutet, n eine Zahl von 2 bis 20, l und k eine Zahl von 2 bis 6, m eine Zahl von 1 bis 40,
R¹¹ C₁-C₂₀-Alkyl, C₂-C₂₀-Dialkylamino-C₂-C₁₀-alkyl, C₁-C₁₀-Alkoxy- C₂-C₁₀-alkyl, C₂-C₂₀-Hydroxyalkyl, C₃-C₁₂-Cycloalkyl, C₄-C₂₀-Cycloalkyl-alkyl, C₂-C₂₀-Alkenyl, C₄-C₃₀-Dialkylamino-alkenyl, C₃-C₃₀-Alkoxyalkenyl, C₃-C₂₀-Hydroxyalkenyl, C₅-C₂₀-Cycloalkyl-alkenyl, Aryl oder C₇-C₂₀-Aralkyl, die unsubstituiert oder durch C₁-C₈-Alkyl, C₂-C₈-Dialkylamino, C₁-C₈-Alkoxy, Hydroxy, C₃-C₈-Cycloalkyl, C₄-C₁₂-Cycloalkyl-alkyl substituiert sein können, oder zwei dieser Substituenten gemeinsam eine gegebenenfalls durch Stickstoff oder Sauerstoff unterbrochene Alkylenkette wie Ethylenoxid, Propylenoxid, Butylenoxid und -CH₂-CH(CH₃)-O- bedeuten.

Gegenstand der Erfindung sind weiterhin Komplexe der oben definierten Verbindungen mit Cobalt, Mangan, Eisen, Ruthenium, Vanadin, Molybdän oder Wolfram. Bevorzugt sind Mangan-Komplexe. Diese Verbindungen sowie die entsprechenden Metallkomplexe eignen sich als Bleich- und Oxidationskatalysatoren bei Persauerstoffverbindungen, insbesondere in solche Persauerstoffverbindungen enthaltenden Wasch- und Reinigungsmitteln, beispielsweise Vollwaschmittel oder Maschinengeschirrspülmittel. Diese Katalysatoren verbessern die Oxidations- und Bleichwirkung der anorganischen Persauerstoffverbindungen bei Temperaturen unterhalb 80°C, insbesondere im Temperaturbereich von 15 bis 45°C bei gleichzeitiger Verringerung der Farb- und Textilfaserschädigung. Darüber hinaus können die oben definierten Verbindungen und deren Metallkomplexe auch in der Papierbleiche eingesetzt werden.

Die Herstellung der Poly-Salen-Dendrimere der Formel I wurde nach einer in der Fachliteratur beschriebenen Methode durchgeführt (R. Moors, F. Vögtle, Chem. Ber. 1993, 126, 2133-2135). Als Initiatorkern dient hierbei Ethylendiamin, das durch eine Michael-Addition mit Acrylnitril umgesetzt wird. Die endständigen Nitrilgruppen werden zum Amin reduziert, wodurch eine weitere Addition von Acrylnitril ermöglicht wird. Durch Wiederholung dieser Synthesefrequenz verdoppelt sich die Zahl der Funktionalitäten. Auf jeden dieser Synthesestufen können die Aminogruppen mit Salicylaldehyd umgesetzt werden, wodurch man Verbindungen der Formel I mit der Gruppe A erhält. Durch Umsetzung der Aminogruppen mit α,β-Diaminopropionsäure und anschließende Reaktion mit Salicylaldehyd gelangt man zu den Verbindungen der Formel I, die Gruppen der Formel -COCHNA-CH₂NA enthalten.

Die Produkte fallen dabei als gelbe Feststoffe bzw. Öle an.
Die Komplexierung mit Metallkationen kann auf drei unterschiedliche Arten erfolgen. Bei der ersten Methode wird der Ligand wie von Moors und Vögtle beschrieben hergestellt. Anschließend erfolgt in einem geeigneten Lösungsmittel, z.B. Chloroform, Methylenchlorid, Ethanol, Methanol, Dimethylformamid, Wasser, Dimethylsulfoxid oder deren Gemische, die Reaktion mit dem Metallkation, beispielsweise zu dem dendritischen Komplex.
In einer zweiten Ausführungsform werden in einer Eintopf-Reaktion Salicylaldehyd, dendritisches Polyamin und Metallsalz, in einem geeigneten Lösungsmittel, z.B. Chloroform, Methylenchlorid, Ethanol, Methanol, Dimethylformamid, Wasser, Dimethylsulfoxid oder deren Gemische zusammengegeben, wodurch die erfindungsgemäßen Katalysatoren gebildet werden.
In der dritten Ausführungsform kann schon das metallfreie Poly-Salen Dendrimer eingesetzt werden. Für diesen Fall nimmt bei der Anwendung das Dendrimer die in dem Wasser befindlichen Metallkationen auf und wirkt als Katalysator. Es ist auch möglich, in einer Waschmittelformulierung das metallfreie Poly-Salen Dendrimer, gegebenenfalls in einer Matrix eingeschlossen, und ein geeignetes Metallsalz getrennt anzuwenden. Durch das Auflösen der Waschmittelformulierung können die Reaktionspartner zueinander gelangen und den Katalysator bilden.

Bevorzugt sind die Verbindungen der Formel I, die Gruppen A enthalten. Besonders bevorzugt sind die Verbindungen bzw. deren Metallkomplexe 4-Kaskade:ethylendiamin[4]:(1-azabutyliden):2-methinphenol, 8-Kaskade:ethylendiamin[4]:(1-azabutyliden)²:2-methinphenol, 16-Kaskade:ethylendiamin[4]:(1-azabutyliden)³:2-methinphenol, 32-Kaskade:ethylendiamin[4]:(1-azabutyliden)⁴:2-methinphenol,

Bei dendritischen Verbindungen wurde, soweit zweckmäßig, die von Newkome vorgeschlagene Dendrimer-Nomenklatur angewandt [G.R. Newkome, C. Morefield, F. Vögtle in Dendritic Macromolecules,VCH, Weinheim 1996].

Derartige Dendrimere können mit stöchiometrisch unterschiedlichen Mengen an Übergangsmetall beladen werden. Im Maximalfall sind sämtliche Stickstoffatome des Dendrimers mit Übergangsmetall abgesättigt.
Neben den peripheren N-Atomen können auch innere Stickstoffatome des Dendrimer komplexieren und der resultierende Komplex katalytisch wirken.
Die Gesamtzahl der peripheren und inneren Stickstoffatome sind:

| | | | | | | | |
|---|---|---|---|---|---|---|---|
| Generation | 0 | 1 | 2 | 3 | 4 | 5 | ... |
| Anzahl N-Atome | 2 | 6 | 14 | 30 | 62 | 126 | |

Die Übergangsmetalle können in den erfindungsgemäß zu verwendenden Komplexen je nach Metall, Oxidationsstufen im Bereich von +II bis +V aufweisen. Mangan, Cobalt und Molybdän gehören zu den bevorzugten Übergangsmetallen. Auch mehrkernige Systeme mit gemischten Oxidationszahlen und/ oder mehreren verschiedenen Übergangsmetallen sind möglich.
Außer dem Dendrimerliganden können die erfindungsgemäß zu verwendenden Komplexverbindungen noch weitere, in der Regel einfacher aufgebaute Liganden, insbesondere Neutral- bzw. ein- oder mehrwertige Anionliganden, tragen. In Frage kommen beispielsweise Wasser, Nitrat, Acetat, Formiat, Citrat, Perchlorat und die Halogenide wie Chlorid, Bromid und Jodid sowie komplexe Anionen wie Hexafluorophosphat. Die Anionliganden sorgen für den Ladungsausgleich zwischen Übergangsmetallzentrum und dem Ligandensystem. Auch die Anwesenheit von Oxo-Liganden, Peroxo-Liganden und Imino-Liganden ist möglich. Diese zusätzlichen Liganden können auch verbrückend wirken, so dass oligomere mehrkernige Komplexe mit mindestens einem Dendrimer-Liganden entstehen.

Die erfindungsgemäßen Übergangsmetall-Dendrimerkomplexe, aber auch die Dendrimere als solche eignen sich hervorragend als Bleich- und Oxidationskatalysatoren, insbesondere in Wasch- und Reinigungsmitteln zum Reinigen von Textilien wie auch von harten Oberflächen, insbesondere von Geschirr und bei der Textil- und Papierbleiche.

Die Wasch- und Reinigungsmittel enthalten diese Bleichkatalysatoren in den Gewichtsmengen von 0,0001 bis 0,5 Gew.-%, insbesondere 0,00025 bis 0,25 Gew.-%, vor allem 0,0005 bis 0,1 Gew.-%, bezogen auf das Gewicht der Formulierungen.

Nachfolgende Beispiele sollen die Erfindung näher erläutern ohne sie darauf einzuschränken.

### Beispiel 1 [4-Kaskade: ethylenediamin [4]:(1-azabutyliden):2-methinphenol] Mangan

In einer Suspension aus 100 ml Toluol und 30 g Na₂SO₄ wurde 5,16 g (43 mmol) Salicylaldehyd gelöst. Hierzu tropfte man in einem Zeitraum von 1 h 3,05 g (10,6 mmol) des zuvor in Toluol suspendierten 4-Kaskade:ethylendiamin[4]:3-propylamin. Es wurde noch 24 h bei Raumtemperatur gerührt und anschließend abfiltriert. Das Lösungsmittel wurde im Vakuum entfernt.

1,3 g der so erhaltenen Verbindung (1,84 mmol) wurden mit 900 mg (3,67 mmol) Mangandiacetat in 50 ml Ethanol 6 h unter Rückfluss erhitzt. Die Reaktionslösung wurde auf ca. 15 ml eingeengt, über Nacht im Kühlschrank stehen gelassen, das Lösungsmittel vollständig entfernt und der Rückstand wurde mit ca. 10 ml MeOH aufgenommen. Der Komplex kristallisiert aus der Lösung als brauner Feststoff (Ausbeute: 1,8 g) aus.

### Beispiel 2: [8-Kaskade:ethylenediamin [4]:(1-azabutyliden)²:2-methinphenol] Mangan

In einer Suspension aus 50 ml Toluol und 15 g Na₂SO₄ löste man 2,2 g (18 mmol) Salicylaldehyd. Hierzu tropfte man in einem Zeitraum von 1 h 1,53 g (2,05 mmol) des zuvor in 50 ml Toluol suspendierten Octaamins (CH₂N(CH₂CH₂N(CH₂CH₂NH₂)₂)₂)₂, wobei sich die Lösung gelb färbte. Es wurde noch 24 h bei Raumtemperatur gerührt und anschließend abfiltriert. Das Lösungsmittel wurde im Vakuum entfernt und der hochviskose gelbe Rückstand mehrfach mit heißem Methanol gewaschen.

2,93 g der so erhaltenen Verbindung (1,86 mmol) in 150 ml Ethanol wurden zunächst mit 30 ml 0,5 m KOH versetzt und 30 min unter Rückfluss erhitzt. Anschließend wurde Mangandiacetat zugesetzt (4,6 g, 18,77 mmol) 45 min unter Rückfluss erhitzt und abgekühlt. Nach Zugabe von 0,95 g LiCI in 7,5 ml Wasser wird noch 45 min bei Raumtemperatur gerührt.
Der Komplex kristallisiert aus der Lösung als brauner Feststoff (Ausbeute: 3,8 g) aus.

## Patentansprüche

1. Verbindungen der Formel I sowie deren Metallkomplexe mit Übergangsmetallen
(R¹R¹)N - X - N(R¹R¹) (I)
worin
R¹ eine Gruppe der Formel (R²R³)N-(CH₂)ₙ-, n die Zahlen 2 oder 3,
R² und R³ jeweils eine Gruppe der Formel (R⁴R⁵)N-(CH₂)ₙ-, n die Zahlen 2 oder 3, oder R² Wasserstoff und R³ eine Gruppe der Formel 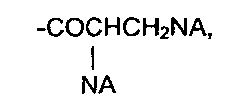
R⁴ und R⁵ jeweils eine Gruppe der Formel (R⁶R⁷)-N-(CH₂)ₙ-, n die Zahlen 2 oder 3 oder R⁴ und R⁵ zusammen die Gruppe der Formel A oder R⁴ Wasserstoff und R⁵ eine Gruppe der Formel 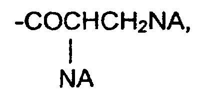
R⁶ und R⁷ jeweils eine Gruppe der Formel (R⁸R⁹)-N-(CH₂)ₙ-, n die Zahlen 2 oder 3 oder R⁶ und R⁷ zusammen die Gruppe der Formel A oder R⁶ Wasserstoff und R⁷ eine Gruppe der Formel 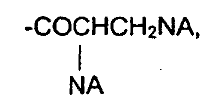
R⁸ und R⁹ zusammen die Gruppe der Formel A oder
R⁸ Wasserstoff und R⁹ eine Gruppe der Formel 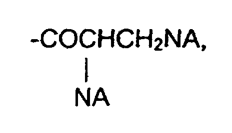
A eine Gruppe der Formel 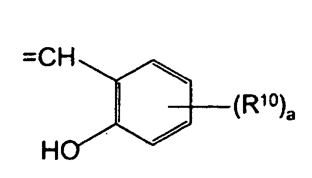
bedeutet, worin a eine ganze Zahl von 1 bis 4 und R¹⁰ Wasserstoff, C₁-C₃₀-Alkyl-, Cycloalkyl- oder Arylreste, C₁-C₄-Alkoxygruppen, substituierte oder unsubstituierte Amino- bzw. Ammoniumgruppen, Halogenatome, Sulfogruppen, Carboxylgruppen oder Gruppierungen der Formel -(CH₂)ᵣ-COOH, -(CH₂)ᵣ-SO₃H, -(CH₂)ᵣ-PO₃H₂, -(CH₂)ᵣ-OH bedeuten, wobei r eine ganze Zahl von 0 bis 4 bedeutet und die genannten Säuregruppen auch in Salzform vorliegen können, und
X eine Gruppe der Formeln
-(CH₂)ₙ-, -(CH₂)₃-NR¹¹-(CH₂)₃-, -(CH₂)₂-NR¹¹-(CH₂)₂-,
-(CH₂)ₗ-[O-(CH₂)ₖ]ₘ-O-CH₂)ₗ)- bedeutet, n eine Zahl von 2 bis 20, l und k eine Zahl von 2 bis 6, m eine Zahl von 1 bis 40,
R¹¹ C₁-C₂₀-Alkyl, C₂-C₂₀-Dialkylamino-C₂-C₁₀-alkyl, C₁-C₁₀-Alkoxy- C₂-C₁₀-alkyl, C₂-C₂₀-Hydroxyalkyl, C₃-C₁₂-Cycloalkyl, C₄-C₂₀-Cycloalkyl-alkyl, C₂-C₂₀-Alkenyl, C₄-C₃₀-Dialkylamino-alkenyl, C₃-C₃₀-Alkoxyalkenyl, C₃-C₂₀-Hydroxyalkenyl, C₅-C₂₀-Cycloalkyl-alkenyl, Aryl oder C₇-C₂₀-Aralkyl, die unsubstituiert oder durch C₁-C₈-Alkyl, C₂-C₈-Dialkylamino, C₁-C₈-Alkoxy, Hydroxy, C₃-C₈-Cycloalkyl, C₄-C₁₂-Cycloalkyl-alkyl substituiert sein können, oder zwei dieser Substituenten gemeinsam eine gegebenenfalls durch Stickstoff oder Sauerstoff unterbrochene Alkylenkette wie Ethylenoxid, Propylenoxid, Butylenoxid und -CH₂-CH(CH₃)-O- bedeuten.

2. Komplexe der Verbindungen der Formel I gemäß Anspruch 1 mit Co, Mn, Fe, Ru, V, Mo oder W.

3. Komplexe der Verbindungen der Formel I gemäß Anspruch 1 mit Mn.

4. Verbindungen der Formel I gemäß Anspruch 1, wobei X eine Gruppe der Formel -(CH₂)ₙ- und n eine Zahl von 2 bis 20 bedeutet.

## Claims

1. A compound of the formula I or a metal complex thereof with a transition metal
(R¹R¹)N - X - N(R¹R¹) (I)
in which
R¹ is a group of the formula (R²R³)N-(CH₂)ₙ-, n is the numbers 2 or 3,
R² and R³ are in each case a group of the formula (R⁴R⁵)N-(CH₂)ₙ-, n is the numbers 2 or 3, or R² is hydrogen and R³ is a group of the formula 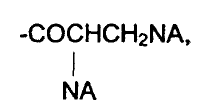
R⁴ and R⁵ are in each case a group of the formula (R⁶R⁷)-N-(CH₂)ₙ-, n is the numbers 2 or 3, or R⁴ and R⁵ together are the group of the formula A or R⁴ is hydrogen and R⁵ is a group of the formula 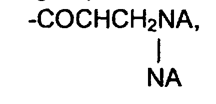
R⁶ and R⁷ are in each case a group of the formula (R⁸R⁹)N-(CH₂)ₙ-, n is the numbers 2 or 3, or R⁶ and R⁷ together are the group of the formula A or R⁶ is hydrogen and R⁷ is a group of the formula 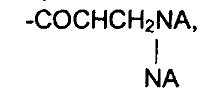
R⁸ and R⁹ together are the group of the formula A or
R⁸ is hydrogen and R⁹ is a group of the formula 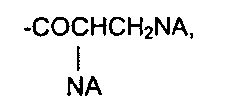
A is a group of the formula 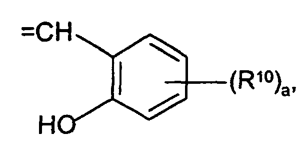
in which a is an integer from 1 to 4, and R¹⁰ is hydrogen, C₁-C₃₀-alkyl, cycloalkyl or aryl radicals, C₁-C₄-alkoxy groups, substituted or unsubstituted amino or ammonium groups, halogen atoms, sulfo groups, carboxyl groups or groups of the formula -(CH₂)ᵣ-COOH, -(CH₂)ᵣ-SO₃H, -(CH₂)ᵣ-PO₃H₂, -(CH₂)ᵣ-OH, where r is an integer from 0 to 4, and said acid groups may also be present in salt form, and
X is a group of the formulae
-(CH₂)ₙ-, -(CH₂)₃-NR¹¹-(CH₂)₃-, -(CH₂)₂-NR¹¹-(CH₂)₂-, -(CH₂)ₗ-[O-(CH₂)ₖ]ₘ-O-CH₂)ₗ)-, n is a number from 2 to 20, l and k are a number from 2 to 6, m is a number from 1 to 40,
R¹¹ is C₁-C₂₀-alkyl, C₂-C₂₀-dialkylamino-C₂-C₁₀-alkyl, C₁-C₁₀-alkoxy-C₂-C₁₀-alkyl, C₂-C₂₀-hydroxyalkyl, C₃-C₁₂-cycloalkyl, C₄-C₂₀-cycloalkyl-alkyl, C₂-C₂₀-alkenyl, C₄-C₃₀-dialkylamino-alkenyl, C₃-C₃₀-alkoxyalkenyl, C₃-C₂₀-hydroxyalkenyl, C₅-C₂₀-cycloalkyl-alkenyl, aryl or C₇-C₂₀-aralkyl which may be unsubstituted or substituted by C₁-C₈-alkyl, C₂-C₈-dialkylamino, C₁-C₈-alkoxy, hydroxyl, C₃-C₈-cycloalkyl, C₄-C₁₂-cycloalkyl-alkyl, or two of these substituents together are an alkylene chain optionally interrupted by nitrogen or oxygen, such as ethylene oxide, propylene oxide, butylene oxide or -CH₂-CH(CH₃)-O-.

2. A complex of the compound of the formula I as claimed in claim 1 with Co, Mn, Fe, Ru, V, Mo or W.

3. A complex of the compound of the formula I as claimed in claim 1 with Mn.

4. A compound of the formula I as claimed in claim 1, where X is a group of the formula -(CH₂)ₙ- and n is a number from 2 to 20.

## Revendications

1. Composés de formule I ainsi que leurs complexes métalliques avec des métaux de transition
(R¹R¹)N-X-N(R¹R¹) (I)
dans lesquels
R¹ représente un groupe de formule (R²R³)N-(CH₂)ₙ-, n le nombre 2 ou 3,
R² et R³ représentent respectivement un groupe de formule (R⁴R⁵)N-(CH₂)ₙ-, n le nombre 2 ou 3,
ou R² représente l'hydrogène et R³ un groupe de formule 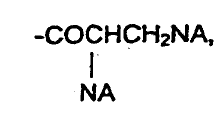
R⁴ et R⁵ représentent respectivement un groupe de formule (R⁶R⁷)N-(CH₂)ₙ-, n le nombre 2 ou 3 ou R⁴ et R⁵ représentent ensemble le groupe de formule A ou R⁴ représente un hydrogène et R⁵ un groupe de formule 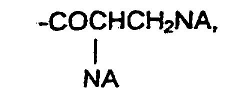
R⁶ et R⁷ représentent respectivement un groupe de formule (R⁸R⁹)N-(CH₂)ₙ-, n le nombre 2 ou 3 ou R⁶ et R⁷ représentent ensemble le groupe de formule A ou R⁶ représente un hydrogène et R⁷ un groupe de formule 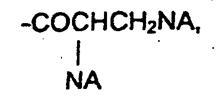
R⁸ et R⁹ représentent ensemble le groupe de formule A ou R⁸ représente l'hydrogène et R⁹ un groupe de formule 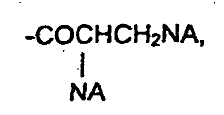 A représente un groupe de formule 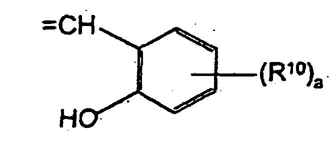 dans laquelle a représente un nombre entier de 1 à 4 et R¹⁰ représente l'hydrogène, des groupes alkyle, cycloalkyle ou aryle en C₁ à C₃₀, des groupes alcoxy en C₁ à C₄, des groupes amino ou ammonium substitués ou non substitués, des atomes d'halogène, des groupes sulfo, des groupes carboxyle ou des groupements de formule -(CH₂)ᵣ-COOH, -(CH₂)ᵣ-SO₃H, -(CH₂)ᵣ-PO₃H₂, -(CH₂)ᵣ-OH, dans lesquelles r représente un nombre entier de 0 à 4 et les groupes acide mentionnés peuvent aussi se présenter sous forme de sels, et
X représente un groupe des formules
-(CH₂)ₙ-, -(CH₂)₃-NR¹¹-(CH₂)₃-, -(CH₂)₂-NR¹¹-(CH₂)₂-, -(CH₂)l-[O-(CH₂)ₖ]ₘ-O-CH₂)ₗ)-, n représente un nombre de 2 à 20, l et k un nombre de 2 à 6, m un nombre de 1 à 40,
R¹¹ représente un alkyle en C₁ à C₂₀, dialkylamino en C₂ à C₂₀-alkyle en C₂ à C₁₀, alcoxy en C₁ à C₁₀-alkyle en C₂ à C₁₀, hydroxyalkyle en C₂ à C₂₀, cycloalkyle en C₃ à C₁₂, cycloalkyle en C₄ à C₂₀-alkyle, alcényle en C₂ à C₂₀, dialkylamino en C₄ à C₃₀-alcényle, alcoxyalcényle en C₃ à C₃₀, hydroxyalcényle en C₃ à C₂₀, cycloalkyle en C₅ à C₂₀-alcényle, aryle ou aralkyle en C₇ à C₂₀, qui peuvent être non substitués ou substitués par un alkyle en C₁ à C₈, dialkylamino en C₂ à C₈, alcoxy en C₁ à C₈, hydroxy, cycloalkyle en C₃ à C₈, cycloalkyle en C₄ à C₁₂-alkyle, ou deux de ces substituants représentent ensemble une chaîne alkylène éventuellement interrompue par un azote ou un oxygène comme l'oxyde d'éthylène, l'oxyde de propylène, l'oxyde de butylène et -CH₂-CH(CH₃)-O-.

2. Complexes des composés de formule I selon la revendication 1 avec Co, Mn, Fe, Ru, V, Mo ou W.

3. Complexes des composés de formule I selon la revendication 1 avec Mn.

4. Composés de formule I selon la revendication 1, dans laquelle X représente un groupe de formule -(CH₂)ₙ- et n un nombre de 2 à 20.
